# EUROPEAN PATENT SPECIFICATION

(11) **EP 0 373 960 B1**
(45) Date of publication and mention of the grant of the patent: **30.07.1997**
(21) Application number: 89313154.0
(22) Date of filing: 15.12.1989
(51) Int. Cl.: C12Q 1/68, C12Q 1/70, C07H 21/04

(54) **Self-sustained, sequence replication system**
4 Selbstfortsetzendes System für Sequenzreplikation
Systéme auto-entretenu de réplication de séquences

(30) Priority: 16.12.1988 US 285467
(43) Date of publication of application: 20.06.1990
(60) Divisional application: 97100006.2
(73) Proprietor: Akzo Nobel N.V., 6824 BM Arnhem (NL)
(72) Inventor: Gingeras, Thomas Raymond, Encinitas California 92024 (US); Guatelli, John C., San Diego California 92117 (US); Whitfield, Kristina Marie, Carlsbad California 92009 (US)
(74) Representative: Baldock, Sharon Claire

(56) References cited:
- EP-A- 0 329 822
- WO-A-88/10315
- THE EMBO JOURNAL, vol. 7, no. 10, October 1988, pages 3289-3297, IRL Press Ltd, Oxford, GB; T. FITZWATER et al.: "Conditional high copy number C0lE1 mutants: resisitance to RNA 1 inhibition in vivo and in vitro"
- SCIENCE, vol. 239, 29th january 1988, pages 491-494; E.S. STOFLET et al.: "Genomic amplification with transcript sequencing"
- NATURE, vol. 333, 30th June 1988, pages 858-860; J. CHELLY et al.: "Transcription of the dystrophin gene in human muscle and non-muscle tissues"
- BIOTECHNOLOGY, vol. 6, October 1988, pages 1197-1202; P.M. LIZARDI et al.: "Exponential amplification of recombinant-RNA hybridization probes"
- PROC. NATL. ACAD. SCI. USA, vol. 87, March 1990, pages 1874-1878; D.C. GUATELLI et al.: "Isothermal, in vitro amplification of nucleic acids by a multienzyme reaction modeled after retroviral replication"

## Description

The present invention relates generally to advances in molecular biology and recombinant DNA technology.

More particularly, the present invention is directed to methods and means, including assays and pharmaceutical kits containing requisite reagents and means, for detecting in an in vitro or ex vivo setting the presence in a biological sample of a target nucleic acid sequence, or of an extrapolated RNA sequence from a corresponding target DNA sequence, and by deduction of corresponding polypeptide that RNA (DNA) sequence encodes.

The present invention features the provision of providing the amplification of such a particular nucleic acid target sequence in a self-sustained, single-pot in vitro system wherein the amplification of the target nucleic acid sequence is accomplished by means of the preparation of multiple transcript products that have the optional capability of self-replication. This self-sustained amplification system avoids the necessity of repeated denaturation of nucleic acid duplexes that require temperature cycling. The present invention combines in a novel manner, all of the reagents necessary to form in a single reaction setting an amplified product, or a product suitable for detection in amplified form, representing the presence of a target nucleic acid sequence.

Among the applications in which the present invention finds use are in analyses of RNA sequences, or by extrapolation, DNA sequences, that are characteristic of a particular or general pathogenic disease or condition by the in vitro or ex vivo nucleic acid probe hybridization assays of body fluids and tissues containing requisite target nucleic acid sequence(s).

It is a goal in this art to detect various nucleic acid sequences in a biological sample, in which a given sequence, a so-called target nucleic acid, is present in small amounts relative to its existence amongst a wide variety of other nucleic acid species including RNA, DNA or both. Thus, it is desirable to detect the nucleic acid encoding polypeptides that may be associated with pathological diseases or conditions, such as, for example, nucleic acid correlating to that of the human immunodeficiency virus (HIV-1). In addition to the detection of nucleic acids encoding such polypeptides, it is desirable to detect other nucleic acids characteristic of a pathological disease or condition such as a defective gene, as in the case of the detection of a defective human betaglobin gene as exemplified in hemophilia.

Characteristically, the nucleic acids associated with such are present, if at all, in very small amounts relative to total nucleic acid in a given biological sample, such as blood or other body fluid or tissue sample of a given individual to be tested. The detection of such nucleic acid species requires such specificity that, if present, it is detectable and measurable from amongst the wide variety of other nucleic acid species with which it is environmentally associated. Some of these species may bear close homology, at least in isolated segments, with the target nucleic acid. Further, as noted above, these target nucleic acid species are very often found only in very minute amounts in the biological sample being tested. And yet, for proper diagnosis of the underlying disease state, it is essential that even small amounts of such target nucleic acid be detectable unequivocally for fidelity of the assay system.

Several approaches have been advanced for accomplishing the goal of the art. In one, the amount of nucleic acid in the sample is not altered or affected. Instead, a reporter system is developed whereby a large number of detectable molecules corresponding to the nucleic acid target are produced for ready detectability and measurement. Such a reporter system is a signal-generating system associated with the target nucleic acid producing a detectable signal representative of the number of molecules of target sequence.

Another approach has been developed that is fundamentally different in that it involves increasing the copy number of the target nucleic acid sequence itself. This can be done by selective amplification of the target nucleic acid sequence. One can refine the culture techniques of the sample such that somehow the target nucleic acid sequence is amplified preferentially to other nucleic acid sequences. These techniques are cumbersome and time consuming and subject to trial and error.

Another example of this approach is amplification of a target nucleic acid sequence in a so-called "polymerase chain reaction" (PCR). This technique was reported by Saiki et al., Science 230, 1350 (1985) and Mullis et al., European Patent Application Publication Nos. 200362 and 201184 (See also U.S. Patents 4683195 and 4683202), and particularly entails (1) hybridizing to a segment of target nucleic acid sequences a primer, (2) extending said primer with a polymerase, and (3) rendering single-stranded the duplexes resulting from the primer extension reaction. This procedure can be repeated over a number of cycles so as to amplify the underlying target nucleic acid sequence.

An improved, novel approach is detailed in PCT International Publication No. WO 88/10315 which forms part of the state of the art by virtue of Article 54(3) EPC. It employs a novel RNA transcript production step in conjunction with, and derived from, a synthesized double-strained cDNA copy of the target sequence operably linked to a promoter therefor. By virtue of the transcription step being the dominant aspect of novelty, it is conveniently referred to as a transcription-based amplification system (TAS).

Thus, that invention involves the in vitro or ex-vivo detection of at least one specific nucleic acid sequence (target sequence or segment) in a sample containing nucleic acid, comprising a method of preparing a double-stranded nucleic acid containing a sequence corresponding to a target sequence operably linked to a RNA-polymerase promoter therefor, employing said double-stranded nucleic acid as a double-stranded nucleic acid template for the preparation of a plurality of RNA transcripts therefrom, each bearing an RNA sequence corresponding to said target sequence, and detecting the presence of said RNA sequence and by analogy the presence of target sequence.

The double-stranded nucleic acid template, in turn, is prepared by providing a first nucleic acid primer or probe containing a promoter sequence operably linked to a sequence corresponding to a segment of a target sequence, hybridizing under suitable conditions said first nucleic acid primer with target sequence in a sample containing nucleic acid, extending the hybridized said first nucleic acid primer in a polymerase extension reaction complementarily to the target sequence to form a corresponding duplex nucleic acid, separating the strands of said duplex, hybridizing to the separated promoter containing sequence strand under suitable conditions a second nucleic acid primer at the end opposite said promoter sequence, and extending the hybridized said second nucleic acid primer in a polymerase extension reaction complementarily to said promoter containing sequence.

Thus, that invention yields a single-stranded RNA transcript, or an RNA-DNA duplex formed therefrom when measures are not undertaken to prevent its formation that has a sequence corresponding to the target nucleic acid. The single-stranded RNA transcript product is struck-off more or less continuously and provides for direct detection of target segment without the necessity of cumbersome, error-prone repeated PCR cycles and strand separation. Such advantages are not provided by the PCR technique that yields double-stranded DNA (one strand of which comprises target segment and the other strand of which comprises complement of target segment) that need to be separated before detection and only after a large number of repeated cycles necessary to reach acceptable amplification levels.

A similar amplification method to that described above is disclosed in EP-A-0329822 in which a first DNA primer is hybridized to a nucleic acid template followed by primer extension and strand separation. A second DNA primer incorporating a promoter is hybridized to the extension product of the first primer and extended to form a double-stranded DNA product which is the template for formation of a plurality of RNA transcripts. EP-A-0329822 also forms part of the state of the art by virtue of Article 54(3) EPC.

It is an object of the present invention to take further advantage of the basic replicative process for amplification, for ease in the detection of target nucleic acid sequences, thus achieving exponential copying without the requirement of temperature cycling and otherwise monitoring the course of the amplification method in respect of reagent additions, etc. It is a further object of the present invention to combine in a novel manner the advantages of the transcription and extension product procedures as a means for detecting and measuring corresponding target nucleic acid.

It is a basic object of the present invention to employ a selective digestion enzymatically of the RNA strand of a RNA/DNA duplex, formed by hybridizing a primer to a nucleic acid target sequence followed by primer extension, as a means of providing the DNA strand as a template for further hybridization thereto followed by primer extension. The product double-stranded DNA duplex contains at least one promoter sequence that is recognizable by a DNA-dependent RNA polymerase and thus serves as a template for the production of a plurality of transcripts that are ultimately detected and measured as a means of detecting and measuring target nucleic acid sequence. This object provides the advantages of target sequence amplification that is self-sustained without the necessity of temperature cycling in a single reaction mixture containing (three) appropriate enzymatic activities and at least one primer containing a promoter operatively recognizable by a DNA-dependent RNA polymerase.

It is thus an overall object of the present invention to meet the goals enumerated by the art and to provide selective means to further advantage amplification of target nucleic acid sequences. It further provides a straightforward technique that can be utilized reproducibly in an acceptably short period of time, in an isothermal reaction system, employing the convenience of known reagents and having the precision necessary to reach consistent scientific results; one that can be employed in a reproducible assay setting and that is adaptable for use in kits for laboratory/clinical analyses.

The present invention is predicated on the use of a means to "strand-separate" a RNA/DNA duplex so as to free the DNA strand thereof for hybridization with obligonucleotides containing RNA polymerase binding sequences (PBS) followed by primer extension so as to form a DNA duplex that can serve as a template for the preparation of plurality of corresponding RNA transcripts which are susceptible to detection and measurement as a deduced assay for the presence of target nucleic acid sequence. The RNA/DNA duplex is in turn produced by hybridization to an RNA target of a DNA primer operatively containing a promoter sequence, followed by primer extension.

The means of the present invention for causing "strand-separation" involves the use of an enzyme having RNase H-like activity, such as RNase H, which will selectively and preferentially digest the RNA strand of the duplex so as to free the DNA strand for further processing. The use of such an enzyme eliminates the use of a temperature cycle to denature said duplex. Further, the products of the selective digestion serve as self-generated primers which can be extended to form a DNA duplex for transcription.

In accordance with the invention there is provided a method of preparing a double-stranded DNA encoding a sequence corresponding to a target RNA sequence and having an operative polymerase promoter, comprising:
a) providing a first DNA primer containing an RNA polymerase promoter sequence operatively associated with a sequence that is a complement of a segment of a target nucleic acid sequence,
b) contacting under suitable hybridizing conditions said first DNA primer with a nucleic acid sample that may contain said target nucleic acid sequence,
c) permitting primer extension of any hybridization product of said first DNA primer with said target nucleic acid sequence in a DNA polymerase extension reaction to form a corresponding RNA/DNA duplex nucleic acid,
d) selectively digesting enzymatically the RNA strand of said RNA/DNA duplex nucleic acid,
e) permitting hybridization to the freed promoter containing cDNA strand under suitable hybridization conditions of a second nucleic acid primer, said second nucleic acid primer being a product of said selective digestion, and
f) permitting primer extension of the hybridization product of the primer with said DNA strand in a DNA polymerase extension reaction.

The method of the invention is useful for the detection of at least one specific target nucleic acid sequence in a nucleic acid sample that may contain said nucleic acid target sequence when comprising the additional step of:
g) employing the prepared double-stranded DNA as described above as a template for the preparation of a plurality of transcripts, each bearing an RNA sequence corresponding to the target nucleic acid sequence.

The nucleic acid target sequence can be one present intrinsically as such in a nucleic acid sample, or it can be a corresponding DNA target sequence extrapolation product. The extrapolation product is prepared by denaturing double-stranded DNA target sequence, hybridizing to it a primer sequence having an operatively associated promoter sequence followed by a primer extension reaction to form a DNA duplex. This DNA/DNA duplex is in turn denatured and the strand containing the promoter sequence hybridized at its end opposite the promoter with a second primer followed by primer extension so as to form a DNA duplex which, when contacted with a DNA-dependent RNA polymerase produces the corresponding transcript, extrapolation product. The RNA then serves as target nucleic acid sequence for purposes of the present invention.

After the nucleic acid target sequence, whatever its source, has been provided, in accordance with the present invention it is hybridized with a primer sequence operatively associated with a promoter sequence, followed by primer extension to produce a RNA/DNA duplex containing a promoter sequence at the 5'-end of the DNA strand. The primer extension reaction can be conducted with any suitable polymerase, such as reverse transcriptase. The basic aspect of the present invention then serves to free the DNA strand of the RNA/DNA duplex by treatment of the RNA/DNA duplex with an enzyme that selectively digests the RNA strand, such as RNase H. The thus freed DNA strand then undergoes self-generated primer extension via anRNA primer resulting from the previous selective digestion. Primer extension creates a double-stranded DNA duplex containing a promoter sequence, that serves as a template for DNA-dependent RNA polymerase induction of transcription to give a plurality of transcripts.

Given that the foregoing reaction sequence can be performed isothermally and in contemporaneous mixture with the three appropriate enzyme activities, such as is provided reverse transcriptase, RNase H and DNA-dependent RNA polymerase, for example, the various steps in the foregoing process are done in a continuous, simultaneous fashion over a given period of time. Thus, at the point provided above as an end point, the produced transcripts can be detected and measured for deduced presence of starting target nucleic acid sequence. However, given the continuous and simultaneous nature of the above-described reaction sequence being independent of temperature cycling and requiring only a single, initial addition of enzyme activities required to carry out these reactions, the transcript products themselves undergo hybridization with a primer optionally bearing operatively an additional promoter sequence. This hybridization complex is followed by primer extension reaction to produce a second RNA/DNA duplex that, in turn, is subjected to the action of the selective RNA digestion enzyme to free the DNA strand therefrom. It is in turn hybridized with a self-generated RNA primer so as to produce a second DNA duplex that is susceptible to recognition by a DNA-dependent RNA polymerase to produce a plurality of transcripts having a sense opposite the transcripts produced initially.

While the mechanism of the foregoing reaction sequence(s) has not been fully elucidated, it is believed that because the reaction mixture employs in combination the three appropriate enzyme activities (such as is provided by reverse transcriptase, RNase H and DNA-dependent RNA polymerase, for example) and at least one primer containing a promoter recognized by the polymerase, and because the reactions are not dependent on a temperature cycle, it is contemplated that where two promoter-containing oligonucleotide primers are used, the reactions may go through several cycles spontaneously and continuously, producing both sense and anti-sense transcripts that may variously be detected and measured to provide an amplification assay of the amount of target nucleic acid sequence present in the sample tested.

The essence of the present invention provides for a reaction mixture that is permitted essentially to remain dormant for a period of time at a suitable temperature, with no need for cycling between higher and lower temperatures and no need for periodic addition of enzyme or other reagents, whereby a target nucleic acid sequence is amplified continuously and spontaneously in a self-generated fashion in the presence of at least one primer bearing operatively a promoter sequence and enzyme activity such as is provided by RNA polymerase that recognizes the polymerase binding site of the promoter, a reverse transcriptase, an enzyme such as RNase H that selectively digests RNA when that RNA is hybridized to DNA in duplex form, and requisite nucleoside triphosphate substrates for the RNA polymerase and reverse transcriptase. In such a system as defined herein, reproducible amplification levels as high as 10⁷ can be achieved in approximately two hours at about 37° C, using for example T7 RNA polymerase, AMV reverse transcriptase and E. coli RNase H. A temperature between about 4° C and about 50° C, preferable in the range around 40° C, is operable. The size of the nucleic acid target sequence in preferred embodiments, contains fewer than about 250 bases. Other variables may affect the optimization of the amplification herein such as the RNA polymerase employed, the reverse transcriptase employed, pHs, salt concentrations, nucleoside triphosphate concentrations. These variables are within the ordinary ken of the skilled artisan.

Thus, the present invention involves the in vitro or ex-vivo detection of at least one specific nucleic acid target sequence in a sample containing a heterogeneous collection of RNAs. The present invention reduces to a method comprising preparing a double-stranded DNA encoding a sequence corresponding to a target sequence and having an operative RNA polymerase promoter, said double-stranded DNA being prepared, in turn, by hybridization followed by primer extension to a DNA strand that has been freed from its RNA complement in a RNA/DNA duplex by action of a RNA selective digestion enzyme, said RNA/DNA duplex having been formed by hybridization with a primer bearing operatively a promoter sequence to a target nucleic acid sequence followed by primer extension. The double-stranded DNA serves as a template for the preparation of a plurality of RNA transcripts therefrom, each bearing an RNA sequence corresponding to said target nucleic acid sequence. The presence of said RNA sequence and by deduction the presence of target sequence, can be detected and measured.

The present invention is directed to all associated methods and means to prepare and use such RNA transcripts. In an embodiment, the present invention is directed to the optionally repetitive method of preparing said double-stranded nucleic acid template defined above providing a first nucleic acid primer containing a promoter sequence operatively linked to a sequence corresponding to a segment of a target nucleic acid sequence, hybridizing under suitable conditions said first nucleic acid primer with target nucleic acid sequence in a sample containing nucleic acid, extending the hybridized said first nucleic acid primer in a polymerase extension reaction complementarily to the target sequence to form a corresponding RNA/DNA duplex nucleic acid, enzymatically cleaving the RNA of said RNA/DNA duplex, hybridizing to the freed promoter-containing DNA sequence strand under suitable conditions a second nucleic acid primer which is a product derived RNA primer, at the end opposite said promoter sequence extending the hybridized said second nucleic acid primer in a polymerase extension reaction complementarily to said promoter-containing sequence.

The present invention in a further embodiment is directed to methods and means of employing said double-stranded nucleic acid supra., as a template for the preparation of a plurality of RNA transcripts therefrom in a reaction catalyzed by a DNA-dependent RNA polymerase that recognizes the promoter thereof, and after further optional cycling as described above, detecting and measuring the presence of said RNA transcripts.

The present invention in a further embodiment, is directed to the improvement in the method of amplifying a target nucleic acid sequence, generated as such or as an extrapolation product from a target DNA sequence, comprising the steps of hybridizing with said target sequence a DNA primer having a promoter sequence operatively linked thereto followed by primer extension to give a corresponding RNA/DNA duplex, hybridization of the freed DNA extension product strand bearing the promoter sequence of said duplex with a second primer at the end opposite the promoter sequence followed by primer extension to form a double-stranded DNA template useful for preparation of optionally replicatable RNA transcripts therefrom for detection as such or for recycling as defined above. The improvement comprises freeing the extension product DNA strand bearing the promoter sequence of said RNA/DNA duplex primer by selective enzymatic digestion of the RNA strand of said RNA/DNA duplex.

In an embodiment, the present invention is directed to the product of the process of treating a RNA/DNA duplex RNA, having linked at the 5'-end of the DNA sequence a promoter sequence, with a selective RNA digestion enzyme.

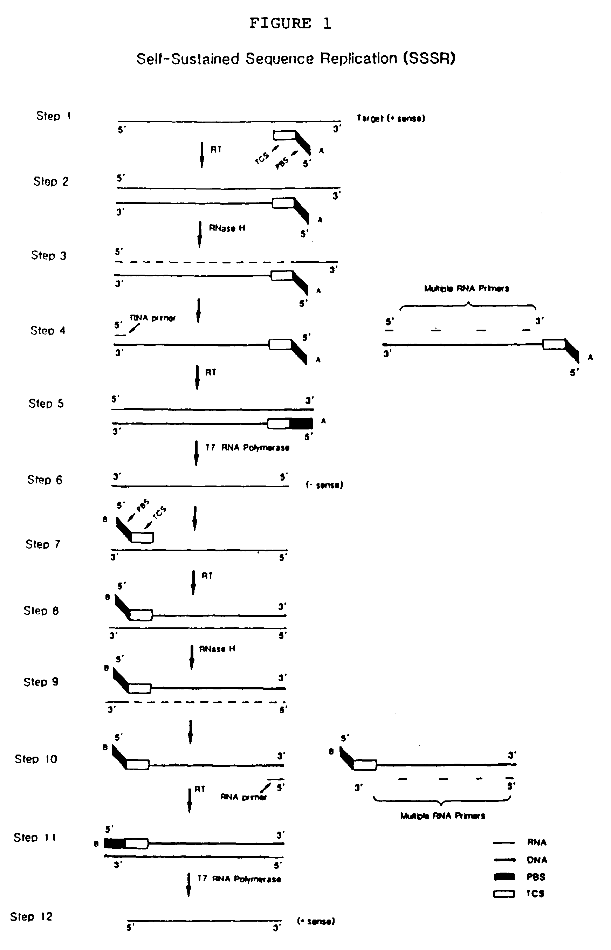

Figure 1 represents a schematic representation of an embodiment of the present invention. The schematic representation provides for a total of 12 steps which, in preferred aspects hereof, are thought to be continuous, self-generated steps upon presence of the requisite three enzymes in the reaction mixture together with target sequence and a given operable temperature. The three enzymes are as listed, e.g., reverse transcriptase (RT) used for primer extension reaction, RNase H which is a selective RNA digestion enzyme, representing the basic aspect of the present invention, and T7 RNA polymerase as an example of a useful enzyme for preparing transcripts from the DNA duplex template. The essence of the invention, as noted above, is represented by step three of the schematic representation and it is understood and contemplated that the RNA transcript product of step six may be detected and measured as such or it may be subjected to continuous reaction as listed in steps 7 through 12 to form the antisense strand of an RNA transcript. The generated RNA transcripts are detected and measured as a consequential result of the presence of the target nucleic acid sequence. PBS represents the polymerase binding site of the promoter sequence. TCS represents target complementary sequence.

Reference is made to standard textbooks of molecular biology that contain definitions and methods and means for carrying out basic techniques of the present invention, such as:
DNA probe or primer preparation, including DNA synthesis or isolation of sequences from natural source via restriction enzyme cleavage and the tailoring thereof so as to be suitable as such or when linked to other DNA for use as a primer or probe herein;
preparation of oligonucleotides with different functional sequences for use in hybridization;
hybridization methodology including variations in stringency conditions for producing more or less hybridization certainty depending on the degree of homology of the primer to a target DNA sequence;
identification, isolation or preparation of promoters, or more specifically promoters or sites recognized by bacteriophage DNA-dependant RNA polymerase and bacteriophage RNA-dependant RNA polymerase or in the employment of eukaryotic systems, viral DNA- and RNA-dependent RNA polymerase, for example, adenovirus-encoded RNA polymerase and brome mosaic virus RNA polymerase;
identification, isolation or preparation of RNA polymerase capable of recognizing said promoters referred to above or capable of primer extension reactions; conditions conducive to the production of RNA transcripts, including so-called transcription-enhancer sequences;
conditions conducive to the initiation and maintenance of primer extension reactions including use of DNA dependent polymerase and dNTPs;
the mechanism and methodology for (induced) replication; and so forth.

See, for example, Maniatis et al., Molecular Cloning: A Laboratory Manual, Cold Spring Harbor Laboratory, New York 1982), and Colowick et al., Methods in Enzymology Vol. 152, Academic Press, Inc. (1987), and the various references cited therein; Hong, Bioscience Reports 1, 243 (1981); Cooke et al., J. Biol. Chem. 255 6502 (1980); and Zoller et al., Methods in Enzymology 100, 468-500 (1983); Crea et al., nucleic acids Res. 8, 2331 (1980); Narang et al., Meth. Enzyme. 68, 90 (1979); Beaucage et al., Tetrahedron Letters 22, 1859 (1981); Brown et al., Meth. Enzym. 68,109 (1979); Caruthers et al., Meth. Enzym. 154, 287 (1985); Hitzeman et al., J. Biol. Chem. 255, 2073 (1980); Lee et al., Science 239, 1288 (1988); Milligan et al., nucleic acids Res. 15, 8783 (1987); Miller et al., Virology 125, 236 (1983), Ahlquist et al., J. Mol. Biol. 153, 23 (1981); Miller et al., Nature 313, 68 (1985); Ahlquist et al., J. Mol. Biol. 172, 369 (1984); Ahlquist et al., Plant Mol. Biol. 3, 37 (1984); Ou et al., PNAS 79, 5235 (1982); Chu et al., Nucl. Acids Res. 14, 5591 (1986); European Patent Application Publn. No. (EPA) 194809; Marsh et al., Positive Strand RNA Viruses, p. 327-336, Alan R. Liss (publ.; New York) 1987; proceedings of UCLA Symposium, 1986); Miller et al., J. Mol. Biol. 187, 537 (1986); Stoflet et al., Science 239, 491 (1988); Kramer et al., J. Mol. Biol. 89, 719 (1974); Saris et al., Nucl. Acids Res. 10, 4831 (1982); Bresser et al., PNAS 80, 6523 (1983); Chu et al., nucleic acids Research 16, 3671 (1988), Gubler et al., Gene 25, 263 (1983) and D'Alessio et al., nucleic acids Res. 16, 1999 (1988), as well as the references cited therein.

All of the aforecited publications are by this reference hereby expressly incorporated by reference herein.

By the term "promoter" is meant a nucleic acid sequence (naturally occurring or synthetically produced or a product of restriction digest) that is specifically recognized by an RNA polymerase that binds to a recognized sequence and initiates the process of transcription whereby an RNA transcript is produced. It may optionally contain nucleotide bases extending beyond the actual recognition site, thought to impart additional stability toward degradation processes, and may also include additional plus (+) nucleotides contiguous to the transcription initiation site. In principle, any promoter sequence may be employed for which there is a known and available polymerase that is capable of recognizing the initiation sequence. Typical, known and useful promoters are those that are recognized by certain bacteriophage polymerase such as bacteriophage T3, T7 or SP6. See Siebenlist et al., Cell 20, 269 (1980). These are but examples of those polymerases that can be employed in the practice of the present invention in conjunction with their associated promoter sequences.

The "RNA transcript" hereof is the ribonucleic acid sequence produced after transcription initiation following RNA polymerase recognition of the promoter sequence (see supra). The production of such transcripts is more or less continuous, dependent in part on the amount of polymerase present.

By the term "probe" or "primer" in the present context is meant a single-stranded nucleic acid sequence (naturally occurring or synthetically produced or a product of restriction digest) that has sufficient complementarity with the target sequence such that under suitable hybridization conditions it is capable of hybridizing, that is binding to, the appropriate (target) sequence. A typical probe or primer is at least about 10 nucleotides in length, and most preferably is of approximately 20 or more nucleotide bases in length, and in its most preferred embodiments, it shares identity or very high complementarity with the appropriate (target) sequence. See, for example, EPA 128042 (publd. 12 Dec 84). Such probe or primer is such that it will hybridize to a complement sequence for purposes of a primer extension reaction in the presence of appropriate reagents and conditions.

The term "operatively linked", or "associated" or grammatical variations thereof, in particular in connection with the linkage of a promoter sequence within an RNA encoding DNA sequence, refers to its functionality in producing corresponding RNA transcripts when the promoter is recognized by the suitable polymerase--see supra.

The techniques of forming a detection signal such as via radioactive labeling or chromogenic means using a chromogenic susceptible enzyme are also well known and documented in the art.

A sample on which the assay method of the invention is carried out can be a raw specimen of biological material, such as serum or other body fluid, tissue culture medium or food material. More typically, the method is carried out on a sample which is a processed specimen, derived from a raw specimen by various treatments to remove materials that would interfere with detection of target, such as by causing non-specific binding of affinity molecules. Methods of processing raw samples to obtain a sample more suitable for the assay methods of the invention are well known in the art.

The transcripts (RNA) can be detected in a number of different ways:

Detection can be by ultraviolet absorbance of RNA, as, for example, by the method of contact photoprinting (Kutateladze et al., Anal. Biochem. 100, 129 (1979)).

By employing a radioactively labeled ribonucleoside-5'-triphosphate in the reaction (e.g., ³H-labeled or alpha-³²PO₄-labeled), so that the RNA is radioactive, the RNA can be detected, by any of numerous known procedures, by means of its radioactivity.

Biotin or iminobiotin can be incorporated into RNA, which can then be detected by known techniques with an enzyme-avidin or enzyme-streptavidin adduct, which binds to the RNA-bound biotin and catalyzes production of a conveniently detectable chromogen. Incorporation of biotin or iminobiotin can be accomplished by employing UTP that is biotinylated through a spacer to carbon-5 of the uracil moiety as a substrate for the replicase in the replication reaction. Such UTP's are known compounds. Further, it is known that such UTP's are substrates for QB replicase, and that RNAs which include uracils biotinylated through spacer groups joined to the carbon-5 position, due to use of such UTP's in their synthesis, are templates for QB replicase catalyzed replication.

RNA can be made fluorescent by employing a T4 RNA ligase catalyzed reaction to append nucleotides modified to be fluorescent to the 3'-end of replicative RNA. See Cosstick et al., Nucl. Acids Res. 12, 1791 (1984). The fluorescence of the resulting RNA can be employed to detect the RNA by any of several standard techniques.

Among still other methods that can be used to detect RNA are those wherein a reporter substance, that binds specifically with nucleic acid, is added to the system in which the replication has taken place, or to the medium, such as a positively charged support such as ECTEOLA paper, on which replicated RNA has been isolated, and signal from the reporter substance measured. Such substances include: chromogenic dyes, such as "stains all" (Dahlberg et al., J. Mol. Biol. 41, 139 (1969); methylene blue (Dingman et al., Biochemistry 7, 659 (1968), and silver stain (Sammons et al., Electrophoresis 2, 135 (1981); Igloi, Anal. Biochem. 134, 184 (1983); fluorogenic compounds that bind to RNA -- for example, ethidium bromide (Sharp et al., Biochemistry 12, 3055 (1973); Bailey et al., Anal. Biochem. 70, 75 (1976); and fluorogenic compounds that bind specifically to RNAs that are templates for replication by QB replicase -- for example, a phycobiliprotein (Oi et al., J. Cell Biol. 93, 981 (1982); Stryer et al., U.S. Patent No. 4,520,110) conjugated to the viral subunit of QB replicase.

In assays according to the invention, assays are carried out simultaneously under conditions as nearly alike as possible, on both test and control samples. As understood in the art, control samples are similar to test samples but are known to contain either no target or a known quantity of target. A control with no target establishes the "background," below which it is not possible to distinguish samples which contain target from those which do not. By comparing the amount or concentration of RNA produced in an assay of a test sample with the amount or concentration produced with control samples assayed simultaneously, the presence of target in test sample at a level above background can be determined. If control samples with a range of known concentrations of target are employed, the concentration of target in a test sample can be estimated.

The use of a "replicase" for (autocatalytic) induction of replication of the optionally replicatable RNA transcripts of the present invention are generally known in the art. Suitable examples of such replicases that are useful in the present invention include the so-called QB virus replicase that recognizes certain nucleic acid sequence sites at the ends of the given RNA transcript and the so-called brome mosaic virus (BMV) as well as the alpha virus replicases which are thought to recognize nucleic acid sequence sites at the 3' end of a given RNA transcript. These replicases serve to replicate, that is reproduce, the RNA transcripts and complements so as to multiply copies thereof. When such enzyme is present in the reaction during the process of transcription, it can be foreseen that the multiple transcripts that are produced during transcription can themselves undergo replication so as to exponentially increase the amount of RNA transcript product.

The quintescense of the present invention is the ability to complete multiple cycles of amplification in vitro without the need for thermal cycling or the addition of supplementary enzymes. The figure appended to the present specification outlines the scheme of a preferred embodiment in diagrammatic fashion. A principal and signal aspect of the present invention is the inclusion of the enzyme RNase H. With further reference to the drawing hereof, steps one and two are identical to those employed in the so-called TAS protocol-cf. the PCT International Application to which reference is made at the outset of this application-but at step three, instead of a thermal denaturation step, the RNA/DNA hybrid duplex is "strand-separated" by the selective digestion of the RNA target by use of RNase H. The RNase H activity has a specificity for RNA only when it is present in an RNA/DNA hybrid duplex. The products of this digestion can either be unique RNA oligomers or multiple RNA oligomers (step 4), and in turn, these oligomers can act as primers for DNA synthesis using reverse transcriptase (RT) as the catalyst of this cDNA reaction (step 5). The double-stranded DNA shown in step 5 can act as a template for T7 RNA polymerase-directed transcription (step 6). This amplified RNA product now serves as detection reporter molecules for target sequence and/or serves as additional target molecules to continue the self-cycling reactions (steps 7 through 12).

The most successful reactions, yielding a ca. 10⁶ fold target amplification, functions with three enzymes and two oligonucleotide primers containing the T7 RNA polymerase binding sequence (PBS). The necessary enzymes are AMV reverse transcriptase, T7 RNA polymerase and E. coli RNase H. Addition of E. coli RNase H in the reaction supplements the RNase activity present in AMV reverse transcriptase and appears necessary to produce high levels of amplification. Selection of optimal oligonucleotide primers centers on the areas of the length of the targeted sequence, inclusion of the polymerase binding sequence on one or both primers and efficiency of the polymerase binding sequence-containing primers as a transcription promoter. All three affect the level of amplification. Inclusion of two oligonucleotide primers, each containing a PBS, resulted in more amplification than did inclusion of a single PBS-containing primer and a non-PBS-containing primer.

With reference to the appended drawing:
(1) mRNA target molecule is annealed with a target specific synthetic oligodeoxyribonucloetide incorporating a T7 RNA polymerase promoter binding sequence,
(2) This primer is extended by the DNA polymerase activity of AMV reverse transcriptase to synthesize the first cDNA strand,
(3) The RNase H activity of AMV reverse transcriptase and E. coli RNase H degrade the RNA of the RNA/DNA hybrid duplex, making the DNA available as a template for second-strand cDNA synthesis,
(4) Self-generated oligoribonucloetides resulting from RNase H digestion prime synthesis of second-strands cDNA. AMV reverse transcriptase then extends the primer to form double-stranded DNA which incorporates an operative T7 RNA polymerase promoter binding sequence,
(5) T7 RNA polymerase binds to the double-stranded promoter binding sequence and transcribes copies of RNA complementary to target nucleic acid,
(6) A second oligodeoxyribonucleotide primer with a PBS anneals to the RNA transcript,
(7) AMV reverse transcriptase catalyses synthesis of a CDNA strand,
(8) RNase H degrades the RNA of the RNA/DNA hybrid duplex and makes the DNA available as a template for second-strand synthesis,
(9) An oligodeoxyribonucleotide primer hybridizes to the second-strand cDNA, and AMV reverse transcriptase synthesizes the DNA. Transcription occurs and cycling continues.

## Claims

1. A method of preparing a double-stranded DNA encoding a sequence corresponding to a target RNA sequence and having an operative polymerase promoter, comprising:
a) providing a first DNA primer containing a RNA polymerase promoter sequence operatively associated with a sequence that is a complement of a segment of a target nucleic acid sequence,
b) contacting under suitable hybridizing conditions said first DNA primer with a nucleic acid sample that may contain said target nucleic acid sequence,
c) permitting primer extension of any hybridization product of said first DNA primer with said target nucleic acid sequence in a DNA polymerase extension reaction to form a corresponding RNA/DNA duplex nucleic acid,
d) selectively digesting enzymatically the RNA strand of said RNA/DNA duplex nucleic acid,
e) permitting hybridization to the freed promoter containing cDNA strand under suitable hybridization conditions of a second nucleic acid primer, said second nucleic acid primer being a product of said selective digestion, and
f) permitting primer extension of hybridization product of primer with said DNA strand in a DNA polymerase extension reaction.

2. A method according to claim 1 useful for the detection of at least one specific target nucleic acid sequence in a nucleic acid sample that may contain said nucleic acid target sequence, comprising the additional step:
g) employing the prepared double-stranded DNA of Claim 1 as a double-stranded DNA template for the preparation of a plurality of RNA transcripts therefrom, each bearing a RNA sequence corresponding to said target nucleic acid sequence.

3. The method according to Claim 2 comprising the additional step of detecting and optionally measuring the presence of said RNA sequence.

4. The method according to any one of Claims 1, 2, or 3 wherein said target RNA sequence in said nucleic acid sample is present intrinsically as such or is a corresponding DNA target sequence extrapolation product prepared by transcription from a double-stranded DNA template prepared by polymerase based primer extension of a primer hybridized to a separated DNA strand operatively bearing a promoter sequence prepared from a hybridization/primer extension product of said DNA target sequence with a primer operatively bearing a promoter sequence.

5. The method according to Claim 2 comprising the additional steps of
h) permitting hybridization of said RNA transcripts under suitable hybridization conditions with a DNA primer containing a promoter sequence operatively associated with a sequence that is a complement of a segment of said RNA transcript sequence,
i) permitting primer extension of hybridized product of step h) in a DNA polymerase extension reaction to form a corresponding RNA/DNA duplex nucleic acid,
j) selectively digesting enzymatically the RNA strand of said RNA/DNA duplex nucleic acid of step i),
k) permitting hybridization of the freed promoter containing DNA strand product of step j) under suitable hybridization conditions with a nucleic acid primer, said nucleic acid primer being a product of said selective digestion of step j),
l) permitting primer extension of hybridization product of step k) in a DNA polymerase extension reaction, and
m) employing the double-stranded DNA product of step l) as a double-stranded DNA template for the preparation of a plurality of RNA transcripts therefrom.

6. The method according to Claim 5 wherein the RNA transcript products have a sense opposite those RNA transcript products of Claim 2.

7. The method according to Claim 5 permitted to proceed continuously and spontaneously by presence in the reaction milieu of enzyme activities provided by:
1) a reverse transcriptase, 2) an enzyme having RNase H activity, 3) a DNA-dependent RNA polymerase and 4) at least one oligonucleotide primer sequence operatively bearing a promoter sequence.

8. The method according to Claim 7 conducted substantially isothermally.

9. A method comprising employing the double-stranded nucleic acid according to Claim 1 or step 1) of Claim 5 as a template for the preparation of a plurality of RNA transcripts therefrom in a reaction catalyzed by a polymerase that recognizes the promoter thereof, each bearing a RNA sequence corresponding to said target nucleic acid sequence, and detecting and optionally measuring the presence of said RNA transcripts.

10. The method according to Claim 2 or 9 wherein said transcripts contain RNA replicase recognition site for replication of said transcripts by RNA replicase induction.

11. The method according to claims 2, 9 or 10 wherein the detected RNA sequence of said RNA transcripts is measured in a manner internally standardized with the presence of a known copy number of nucleic acid also contained in said sample.

12. The method according to Claim 1 wherein said target nucleic acid sequence is associated with the characteristics of a genetic or pathogenic disease or condition.

13. The method according to Claim 1 wherein said target nucleic acid sequence is associated with a human immunodeficiency virus.

14. The method according to Claim 1 wherein said target nucleic acid sequence is associated with a defective gene.

15. The method according to Claim 1 or 5 wherein the promoter is a bacteriophage T7 promoter and the RNA transcripts are produced using T7 RNA polymerase.

16. The method according to Claim 1 or 5 wherein the selective digestion is conducted with RNase H enzyme.

17. The method according to Claim 1 or 5 wherein the extension reaction is catalyzed by E. coli DNA polymerase I.

18. The method according to Claim 1 or 5 wherein the extension reaction is catalyzed by Klenow fragment of E. coli DNA polymerase I.

19. The method according to Claim 1 or 5 wherein the extension reaction is catalyzed by T7 DNA polymerase.

20. The method according to Claim 1 or 5 wherein the extension reaction is catalyzed by reverse transcriptase.

21. The method according to Claim 2 or 5 wherein said RNA transcripts are labelled prior to detection.

22. The method according to Claim 21 wherein said RNA transcripts are radio-labelled.

23. The method according to Claim 21 wherein said RNA transcripts are chromophore labelled.

## Patentansprüche

1. Verfahren zur Herstellung einer doppelsträngigen DNA, die eine einer Target-RNA-Sequenz entsprechende Sequenz codiert und einen operativen Polymerasepromotor hat, mit folgenden Schritten:
a) Bereitstellen eines ersten DNA-Primers, der eine RNA-Polymerasepromotorsequenz enthält, die operativ mit einer Sequenz verbunden ist, die ein Komplement eines Segments einer Target-Nucleinsäuresequenz ist,
b) Kontaktieren unter geeigneten Hybridisierungsbedingungen des ersten DNA-Primers mit einer Nucleinsäureprobe, die die Target-Nucleinsäuresequenz enthalten kann,
c) Ermöglichen der Primerextension eines Hybridisienungsprodukts des ersten DNA-Primers mit der Target-Nucleinsäuresequenz in einer DNA-Polymeraseextensionsreaktion zur Bildung einer entsprechenden RNA/DNA-Duplexnucleinsäure,
d) selektives enzymatisches Aufspalten des RNA-Stranges der RNA/DNA-Duplexnucleinsäure,
e) Ermöglichen der Hybridisierung eines zweiten Nucleinsäureprimers an den freigesetzten Promotor, der einen cDNA-Strang enthält, unter geeigneten Hybridisierungsbedingungen, welcher zweite Nucleinsäureprimer ein Produkt der selektiven Aufspaltung ist, und
f) Ermöglichen der Primerextension des Hybridisierungsprodukts des Primers mit dem DNA-Strang in einer DNA-Polymeraseextensionsreaktion.

2. Verfahren nach Anspruch 1, das zum Erfassen mindestens einer spezifischen Target-Nucleinsäuresequenz in einer Nucleinsäureprobe nützlich ist, die die Nucleinsäure-Targetsequenz enthalten kann, mit dem zusätzlichen Schritt:
g) Verwenden der hergestellten doppelsträngigen DNA aus Anspruch 1 als ein doppelsträngiges DNA-Template zur Herstellung einer Vielzahl von RNA-Transkripten daraus, von welchen jedes eine RNA-Sequenz trägt, die der Target-Nucleinsäuresequenz entspricht.

3. Verfahren nach Anspruch 2, mit dem zusätzlichen Schritt des Erfassens und optional des Messens der Anwesenheit der RNA-Sequenz.

4. Verfahren nach einem der Ansprüche 1, 2 oder 3, bei welchem die Target-RNA-Sequenz in der Nucleinsäureprobe von sich aus als solche vorhanden ist oder ein entsprechendes DNA-Targetsequenz-Extrapolierungsprodukt ist, das durch Transkription von einem doppelsträngigen DNA-Template hergestellt wird, das durch eine Primerextension auf Polymerasebasis eines an einen separaten DNA-Strang hybridisierten Primers hergestellt ist, welcher operativ eine Promotorsequenz trägt, die aus einem Hybridisierungs-/Primerextensionsprodukt der DNA-Targetsequenz mit einem Primer, der operativ eine Promotorsequenz trägt, hergestellt ist.

5. Verfahren nach Anspruch 2, mit den zusätzlichen Schritten:
h) Ermöglichen der Hybridisierung der RNA-Transkripte unter geeigneten Hybridisierungsbedingungen mit einem DNA-Primer, der eine Promotorsequenz enthält, die operativ mit einer Sequenz verbunden ist, die ein Komplement eines Segments der RNA-Transkriptsequenz ist,
i) Ermöglichen der Primerextension des hybridisierten Produkts aus Schritt h) in einer DNA-Polymeraseextensionsreaktion zur Bildung einer entsprechenden RNA/DNA-Duplexnucleinsäure,
j) selektives enzymatisches Aufspalten des RNA-Stranges der RNA/DNA-Duplexnucleinsäure aus Schritt i),
k) Ermöglichen der Hybridisierung des freigesetzten, den Promotor enthaltenden DNA-Strangprodukts aus Schritt j) unter geeigneten Hybridisierungsbedingungen mit einem Nucleinsäureprimer, welcher Nucleinsäureprimer ein Produkt der selektiven Aufspaltung aus Schritt j) ist,
l) Ermöglichen der Primerextension des Hybridisierungsprodukts aus Schritt k) in einer DNA-Polymeraseextensionsreaktion, und
m) verwenden des doppelsträngigen DNA-Produkts aus Schritt l) als ein doppelsträngiges DNA-Template zur Herstellung einer Vielzahl von RNA-Transkripten von diesem.

6. Verfahren nach Anspruch 5, bei welchem die RNA-Transkriptprodukte eine den RNA-Transkriptprodukten aus Anspruch 2 entgegengesetzte Drehrichtung haben.

7. Verfahren nach Anspruch 5, dessen kontinuierlicher und spontaner Fortschritt durch die Anwesenheit von Enzymaktivitäten in dem Reaktionsmilieu ermöglicht wird, die bereitgestellt werden durch:
1) eine reverse Transkriptase, 2) ein Enzym, das RNase-H-Aktivität hat, 3) eine DNA-abhängige RNA-Polymerase und 4) mindestens eine Oligonucleotidprimersequenz, die eine Promotorsequenz operativ trägt.

8. Verfahren nach Anspruch 7, welches im wesentlichen isothermisch ausgeführt wird.

9. Verfahren mit Verwendung der doppelsträngigen Nucleinsäure nach Anspruch 1 oder aus Schritt 1) von Anspruch 5 als ein Template zur Herstellung einer vielzahl von RNA-Transkripten daraus in einer Reaktion, die durch eine Polymerase katalysiert ist, die deren Promotor erkennt, wobei jede eine RNA-Sequenz trägt, die der Target-Nucleinsäuresequenz entspricht, und Erfassen und optional Messen der Anwesenheit der RNA-Transkripte.

10. Verfahren nach Anspruch 2 oder 9, bei welchem die Transkripte eine RNA-Replikaseerkennungsstelle zur Replikation der Transkripte durch RNA-Replikaseinduzierung enthalten.

11. Verfahren nach Anspruch 2, 9 oder 10, bei welchem die erfaßte RNA-Sequenz der RNA-Transkripte in einer Weise gemessen wird, die intern mit der Anwesenheit einer bekannten Kopienzahl von Nucleinsäure, die ebenfalls in der Probe vorhanden ist, standardisiert ist.

12. Verfahren nach Anspruch 1, bei welchem die Target-Nucleinsäuresequenz mit den Charakteristiken einer genetischen oder pathogenen Erkrankung oder eines solchen Leidens in Zusammenhang steht.

13. Verfahren nach Anspruch 1, bei welchem die Target-Nucleinsauresequenz mit einem menschlichen Immunmangelvirus in zusammenhang steht.

14. Verfahren nach Anspruch 1, bei welchem die Target-Nucleinsäuresequenz mit einem fehlerhaften Gen in Zusammenhang steht.

15. Verfahren nach Anspruch 1 oder 5, bei welchem der Promotor ein Bakteriophage-T7-Promotor ist und die RNA-Transkripte unter Verwendung einer T7-RNA-Polymerase erzeugt werden.

16. Verfahren nach Anspruch 1 oder 5, bei welchem das selektive Aufspalten mit einem RNase-H-Enzym ausgeführt wird.

17. Verfahren nach Anspruch 1 oder 5, bei welchem die Extensionsreaktion durch eine E.-coli-DNA-Polymerase I katalysiert wird.

18. Verfahren nach Anspruch 1 oder 5, bei welchem die Extensionsreaktion durch das Klenow-Fragment der E.-coli-DNA-Polymerase I katalysiert wird.

19. Verfahren nach Anspruch 1 oder 5, bei welchem die Extensionsreaktion durch die T7-DNA-Polymerase katalysiert wird.

20. Verfahren nach Anspruch 1 oder 5, bei welchem die Extensionsreaktion durch eine reverse Transkriptase katalysiert wird.

21. Verfahren nach Anspruch 2 oder 5, bei welche die RNA-Transkripte vor der Erfassung markiert werden.

22. Verfahren nach Anspruch 21, bei welchem die RNA-Transkripte radioaktiv markiert werden.

23. Verfahren nach Anspruch 21, bei welchem die RNA-Transkripte Chromophor-markiert werden.

## Revendications

1. Procédé de préparation d'un ADN double brin codant pour une séquence correspondant à une séquence d'ARN cible et ayant un promoteur de polymérase fonctionnel, comprenant :
a) l'obtention d'une première amorce d'ADN contenant une séquence promotrice d'ARN-polymérase liée de manière fonctionnelle à une séquence qui est complémentaire d'un segment d'une séquence d'acide nucléique cible,
b) la mise en contact dans des conditions d'hybridation convenables de ladite première amorce d'ADN avec un échantillon d'acide nucléique susceptible de contenir ladite séquence d'acide nucléique cible,
c) l'extension d'amorce de tout produit d'hybridation de ladite première amorce d'ADN à ladite séquence d'acide nucléique cible dans une réaction d'extension médiée par ADN-polymérase en vue de former un duplex d'acide nucléique ARN/ADN correspondant,
d) la digestion enzymatique sélective du brin d'ARN dudit duplex d'acide nucléique ARN/ADN,
e) l'hybridation au brin d'ADNc libéré contenant un promoteur dans des conditions d'hybridation convenables d'une deuxième amorce d'acide nucléique, ladite deuxième amorce d'acide nucléique étant un produit de ladite digestion sélective, et
f) l'extension d'amorce du produit d'hybridation de l'amorce audit brin d'ADN dans une réaction d'extension médiée par ADN-polymérase.

2. Procédé selon la revendication 1, utile pour la détection d'au moins une séquence d'acide nucléique cible spécifique dans un échantillon d'acide nucléique susceptible de contenir ladite séquence cible d'acide nucléique, comprenant en outre l'étape :
g) d'utilisation de l'ADN double brin préparé selon la revendication 1 comme matrice d'ADN double brin pour en préparer une pluralité de transcrits d'ARN, chaque transcrit portant une séquence d'ARN correspondant à ladite séquence d'acide nucléique cible.

3. Procédé selon la revendication 2, comprenant en outre l'étape de détection et de mesure, éventuellement, de la présence de ladite séquence d'ARN.

4. Procédé selon l'une quelconque des revendications 1, 2 ou 3, dans lequel ladite séquence d'ARN cible dans ledit échantillon d'acide nucléique est présente de manière intrinsèque comme telle ou est un produit d'extrapolation de la séquence cible d'ADN correspondante, préparé par transcription d'une matrice d'ADN double brin obtenue par extension d'amorce médiée par polymérase d'une amorce hybridée à un brin d'ADN dissocié portant une séquence promotrice fonctionnelle, lequel est préparé à partir d'un produit d'hybridation/extension d'amorce de ladite séquence cible d'ADN à une amorce portant une séquence promotrice fonctionnelle.

5. Procédé selon la revendication 2, comprenant en outre les étapes
h) d'hybridation desdits transcrits d'ARN dans des conditions d'hybridation appropriées à une amorce d'ADN contenant une séquence promotrice liée de manière fonctionnelle à une séquence qui est complémentaire d'un segment de ladite séquence de transcrit d'ARN,
i) d'extension d'amorce du produit d'hybridation de l'étape h) dans une réaction d'extension médiée par ADN-polymérase en vue de former un duplex d'acide nucléique ARN/ADN correspondant,
j) de digestion enzymatique sélective du brin d'ARN dudit duplex d'acide nucléique ARN/ADN obtenu à l'étape i),
k) d'hybridation du brin d'ADN produit libéré contenant un promoteur de l'étape j) à une amorce d'acide nucléique dans des conditions d'hybridation appropriées, ladite amorce d'acide nucléique étant un produit de ladite digestion sélective de l'étape j),
l) d'extension d'amorce du produit d'hybridation de l'étape k) dans une réaction d'extension médiée par ADN-polymérase, et
m) d'utilisation de l'ADN double brin produit à l'étape l) comme matrice d'ADN double brin pour en préparer une pluralité de transcrits d'ARN.

6. Procédé selon la revendication 5, dans lequel les produits transcrits d'ARN sont orientés en sens inverse par rapport aux produits transcrits d'ARN de la revendication 2.

7. Procédé selon la revendication 5 à déroulement continu et spontané grâce à la présence dans le milieu réactionnel d'activités enzymatiques assurées par :
1) une transcriptase inverse, 2) une enzyme douée d'activité RN-ase H, 3) une ARN-polymérase ADN-dépendante et 4) au moins une séquence d'amorce oligonucléotidique portant une séquence promotrice fonctionnelle.

8. Procédé selon la revendication 7 exécuté essentiellement en milieu isotherme.

9. Procédé comprenant l'emploi d'acide nucléique double brin selon la revendication 1 ou l'étape 1) de la revendication 5 comme matrice pour en préparer une pluralité de transcrits d'ARN dans une réaction catalysée par une polymérase qui reconnaît le promoteur de tels transcrits, chaque transcrit portant une séquence d'ARN correspondant à ladite séquence d'acide nucléique cible, et la détection et la mesure, éventuellement, de la présence desdits transcrits d'ARN.

10. Procédé selon la revendication 2 ou 9, dans lequel lesdits transcrits contiennent un site de reconnaissance d'ARN-réplicase pour la réplication desdits transcrits par induction de l'ARN-réplicase.

11. Procédé selon les revendications 2, 9 ou 10 dans lequel la séquence d'ARN desdits transcrits d'ARN détectée est mesurée selon un mode à étalonnage interne en présence d'un nombre de copies connu d'acide nucléique contenu également dans ledit échantillon.

12. Procédé selon la revendication 1, dans lequel ladite séquence d'acide nucléique cible est associée aux caractéristiques d'une affection ou d'une maladie à agent pathogène ou génétique.

13. Procédé selon la revendication 1, dans lequel ladite séquence d'acide nucléique cible est associée à un virus de l'immunodéficience humain.

14. Procédé selon la revendication 1, dans lequel ladite séquence d'acide nucléique cible est associée à un gène défectueux.

15. Procédé selon la revendication 1 ou 5, dans lequel le promoteur est un promoteur de bactériophage T7 et les transcrits d'ARN sont produits en utilisant l'ARN-polymérase de T7.

16. Procédé selon la revendication 1 ou 5 dans lequel la digestion sélective est conduite par l'enzyme RN-ase H.

17. Procédé selon la revendication 1 ou 5, dans lequel la réaction d'extension est catalysée par l'ADN-polymérase I d'E. coli.

18. Procédé selon la revendication 1 ou 5, dans lequel la réaction d'extension est catalysée par le fragment de Klenow de l'ADN-polymérase I d'E. coli.

19. Procédé selon la revendication 1 ou 5, dans lequel la réaction d'extension est catalysée par l'ADN-polymérase de T7.

20. Procédé selon la revendication 1 ou 5, dans lequel la réaction d'extension est catalysée par la transcriptase inverse.

21. Procédé selon la revendication 2 ou 5, dans lequel lesdits transcrits d'ARN sont marqués préalablement à leur détection.

22. Procédé selon la revendication 21, dans lequel lesdits transcrits d'ARN sont radiomarqués.

23. Procédé selon la revendication 21, dans lequel lesdits transcrits d'ARN sont marqués par des chromophores.
